# EUROPEAN PATENT APPLICATION

(11) **EP 4 400 096 A1**
(43) Date of publication of application: **17.07.2024**
(21) Application number: 23208680.1
(22) Date of filing: 09.11.2023
(51) Int. Cl.: A61K 9/06, A61K 47/44, A61K 31/01, A61K 31/00

(54) **TOPICAL COMPOSITION CONTAINING CANNABINOIDS**

(30) Priority: 09.11.2022 US 202263423968 P
(71) Applicant: TF Holdings LLC, Aurora, CO 80010 (US)
(72) Inventor: Fanning, Francis G., Jackson, 83001 (US); Ripsom, Tobias C., Denve, 80218 (US); Peterson, Marnie, L., Jackson, 83001 (US); Onyebuagu, William, South Holland, 60473 (US)
(74) Representative: AWA Sweden AB

(57) **Abstract**

A topical composition for skin ailments, conditions or diseases comprising lanolin or derivatives thereof, one or more extracted vegetable oils, one or more extracted plant oils, and one or more cannabinoids. The cannabinoids used in the topical composition may be selected cannabinoids such as CBD and/or analogs and derivatives, THC and/or analogs and derivatives, or mixtures thereof. The extracted vegetable oils and plant oils used in the topical composition may be squalane, meadowfoam seed oil, or a mixture thereof. These components used in the topical composition may be naturally derived from vegetables, plants, or animals. The topical composition has effective pharmacological activity to care for or treat skin ailments, conditions or diseases and may be used to care for or treat a human or an animal.

## Description

### BACKGROUND

There are many different types of skin ailments, conditions or diseases that vary greatly in symptoms and severity. Although some skin ailments, conditions or diseases are minor; others can be life-threatening.

The pharmaceutical and cosmetic industries have brought many products to market to address a plethora of skin ailments, conditions and diseases. These products include, for example, formulations for topical administration that are directly applied to external body surfaces such as the skin or mucous membranes to treat skin or mucous membranes using a large range of formulation classes including creams, foams, gels, lotions, and ointments.

In a selected group of pharmaceutical and cosmetic products containing cannabinoids are the subject of much research in the pharmaceutical and cosmetic industries. These and other cannabinoid-containing compositions provide treatments for many skin disorders. However, many of these types of compositions and methods suffer from significant limitations and do not treat all types of skin disorders or problems.

Thus, there exists a need for new cannabinoid-containing compositions, formulations and methods to use cannabinoids compositions to better treat skin ailments, conditions or diseases.

### SUMMARY

This disclosure provides embodiments of a topical composition for use on skin ailments, conditions or diseases comprising a combination of a pharmacologically effective amount of one or more extracted vegetable oils, one or more extracted plant oils, and one or more cannabinoids.

One embodiment is a therapeutic topical composition comprising pharmacologically effective amounts of meadowfoam seed oil, squalane, and a cannabinoid or mixtures thereof.

In still other embodiments, varying amounts of extracted vegetable oils are used. Suitable amounts of extracted vegetable oils, such as squalane, include ranges of about 1-25 wt%, about 5-20 wt%, about 1-10 wt%, about 2-8 wt%, about 3-7 wt%, or about 1-5 wt%.

In other embodiments, varying amounts of extracted plant oils are used. Suitable amounts of extracted plant oils, such as meadowfoam seed oil, include ranges of about 1-20 wt%, about 4-15 wt%, about 2-10 wt%, about 4-8 wt%, or about 5-7 wt%.

In some embodiments, varying amounts of cannabinoids are used. A person skilled in the art would understand and select amounts of cannabinoids that provide a beneficial biological or clinical activity and a therapeutic window for treating specific or particular skin ailments, conditions or diseases. Suitable amounts of cannabinoids include, for examples, ranges of about 0.05-15 wt%, about 0.25-10 wt%, about 0.25-5 wt%, about 0.5-5 wt%, about 1-5 wt%, or about 1-3 wt%. These cannabinoids may comprise, for example, cannabidiol (CBD including all cannabidiol derivatives), delta-9-tetrahydrocannabinol (THC including all delta-9-tetrahydrocannabinol derivatives), or both CBD and THC. In some embodiments, the CBD comprises about 0.25-10 wt% of the total composition. In other embodiments, the topical composition comprises about 0.25-5 wt% CBD and about 0.25-5 wt% THC.

In some embodiments the topical composition comprises (i) about 0.05-5 wt% by weight of at least one cannabinoid; and (ii) about 1-4 wt% by weight of at least one extracted vegetable oil and one extracted plant oil.

In other embodiments of the topical composition the components comprise one or more of the following features:
(a) the cannabinoid, the extracted vegetable oil, and extracted plant oil comprise a naturally derived product;
(b) the cannabinoid comprises CBD, THC, or CBD and THC;
(c) the CBD comprises about 0.25-5 wt% by weight of the total composition;
(d) the composition comprises about 0.25-5% by weight of CBD and about 0.25-5% by weight of THC;
(e) the extracted vegetable oil and extracted plant oil comprise meadowfoam seed oil and squalane;
(f) the composition comprises about 5-20 wt% of meadowfoam seed oil and about 4-15 wt% of squalane; or
(g) the composition comprises about 0.25-3% by weight of CBD, about 0.25-3% by weight of THC, about 5-20 wt% of meadowfoam seed oil, and about 4-15 wt% of squalane.

Other embodiments of the topical composition described in this disclosure may comprise other components comprising, but not limited to, one or more of a solvent, a thickener, an anti-oxidant, a conditioner, a moisturizer, an emulsifier, a nutrient, an aroma or fragrance, a preservative, a stabilizer, or a combination thereof. These topical compositions may be in the different application forms comprising, for example, an ointment, a cream, an emulsion, a lotion, a paste, an unguent, or a gel, and alternatively, may comprise a waterproof shield on the skin.

A particular embodiment described is a topical composition comprising a pharmacologically effective antimicrobial composition comprising pharmacologically effective amounts of meadowfoam seed oil, squalane and cannabinoids; wherein the cannabinoids topically penetrate and treat the skin. A "pharmacologically effective amount" comprises an amount effective to bring about a biological reaction, physiological reaction, a physical change, or a desired clinical benefit or outcome on skin of a human or animal. Determination of an effective amount is typically within the capacity of persons skilled in the art, especially in light of this disclosure. A "pharmacologically effective amount" will be an amount that provides the desired effect when topically applied to care for or treat skin ailments, conditions or diseases.

This disclosure also provides a method of caring for or treating skin ailments, conditions or diseases by applying a therapeutically effective amount of the embodied topical compositions in this disclosure to the skin of a human or an animal in need of care or treatment and the embodied topical composition comprises one or more cannabinoids that will topically penetrate skin in order to care for or treat the skin.

### DETAILED DESCRIPTION

This disclosure describes embodiments of a topical composition to care for or treat skin ailments, conditions or diseases comprising one or more extracted vegetable oils, one or more extracted plant oils, and one or more cannabinoids.

### Extracted Vegetable Oils and Plant Oils

This disclosure describes an enhanced efficacy of one or more cannabinoids in a topical composition by formulating them with extracted vegetable oils and plant oils. In one embodiment, pharmacological properties of cannabinoids, such as CBD or THC, alone or together, can be enhanced by combining them with an extracted vegetable oils and plant oils such as, for example, meadowfoam seed oil and squalane. These components may provide beneficial properties and may be used as an emollient, a penetration enhancer, a dispersion agent of the cannabinoids, or a combination of these properties.

Naturally derived squalane, such as hydrogenated squalene, is highly stable to oxidation. It can be found in olives and other vegetable-derived substances, such as, for example, wheat germ. Due to its stability, emollience, and moisturizing effects, squalane can play a valuable role in maintaining the skin barrier film, thereby preventing trans epidermal moisture loss. Squalane is a hydrocarbon that may be derived by hydrogenation of squalene. In contrast to squalene, due to the complete saturation of squalane, it is not subject to autooxidation.

Squalene was traditionally sourced from the livers of sharks. The hydrogenation of squalene to produce squalane was first reported in 1916. Now, other sources such as plant sources including, but not limited to, olive oil, rice and sugar cane have been used to commercially produce squalane. In addition, recent advances in microbial engineering have reported making cell-based squalane from a number of microbes or microbial sources. In one example, microbes have also been used to produce squalene, such as *Bacillus subtilis,* Song et al, "Production of Squalene in Bacillus subtilis by Squalene Synthase Screening and Metabolic Engineering", J. Agric Food Chem. 2020, 68, 15, 4447-4455. Further examples of these production procedures and processes are reported in Paramasivan et al., "Recent advances in the microbial production of squalene", World Journal of Microbiology and Biotechnology volume 38, Article number: 91 (2022), and Popa et al., " Methods for obtaining and determination of squalene from natural sources", Biomed Res Int. 2015; 2015:367202.

Naturally derived meadowfoam seed oil is a high molecular weight plant oil, which can be expressed from the seeds of the Meadowfoam plant *(Limnanthes* Alba). A typical plant profile is: eicosenoic acid C₂₀: 1-61%; docosenoic acid C₂₂: 1-16%; and docosadienoic acid C₂₂: 2-18%. Meadowfoam seed oil typically contains some phytosterols. One of its properties is that it is a liquid at relatively low and ambient temperatures (*i.e*., room temperatures) and stable at both high and very low temperatures (*i*.*e*., freezing temperatures). In addition, it readily absorbs into the epidermal region of the skin. As a carrier oil, meadowfoam seed oil is non-comedogenic and does not clog skin pores. It can perform as an emollient and skin softener. It is commonly used in skin care products to help balance sebum production. It can also serve as a 'carrier' for an active ingredient into the epidermal region of the skin.

Squalane and meadowfoam seed oil are high molecular weight, stable, naturally derived plant oils that can enhance the penetration of a topical composition to the outer level of the skin. Exemplified embodiments show that a combination of squalane and meadowfoam seed oil provide, in part, a delivery base for CBD and/or THC. In some embodiments these two naturally derived components will 'surround' or 'encase' the molecules of the CBD and the THC to deliver them 'intact' or `stable' until they are released onto the skin. Both of the oils can also function to help penetration of the topical composition to the epidermal region of the skin which can deliver enhanced pharmacological efficacy to the site in lower levels of the skin.

### Cannabinoids

Cannabinoids are the subject of ongoing research in the pharmaceutical and cosmetic industries. Patents and patent applications in this field include US 10,441,552; US 10,835,504; US 10,864,189; US 11,058,646; US 2016/0120803; US 2020/0352849; US 2021/0046040; and EP 3 875 100. These and other publications disclose the use of cannabinoids in compositions to treat various disorders, such as, toe nail fungus, MRSA infection, herpes virus infection, tinea pedis, burns, infections, sun burn, diabetic infection, eczema, impetigo, dermatophytosis, psoriasis, itchy skin, painful skin, inflamed skin, atopic dermatitis, dandruff, pruritus ani, and general topical infections.

In this disclosure suitable cannabinoids include, but are not limited to, natural phytocannabinoids, organic cannabinoids, endocannabinoids, cannabinoid analogs, cannabinoid derivatives, synthetic cannabinoids and cannabinoid receptor agonists. Those skilled in the art know cannabinoids comprise compounds that interact with a cannabinoid receptor and other cannabinoid mimetics. Examples may include, but not limited to, certain tetrahydropyran analogs (delta-9-tetrahydrocannabinol, delta-8-tetrahydrocannabinol, 6,6,9-trimythel-3-pentyl-6H-dibenzo[b,d]pyran-1-ol, 3-(1,1-dimethylheptyl)-6,6a7,8,10,10a-hexahydro-1-1hydroxy-6,6-dimythel-9- H-dibenzo[b,d]pyran-9-ol,(-)--(3 S,4S)-7-hydroxy-delta-6-tetrahydrocannabinol-1,1-dimethylheptyl, (+)--(3 S,4S)-7-hydroxy-A-6-tetrahydrocannabinol, and delta-8-tetrahydrocannabinol-11-oic acid); certain piperidine analogs (e.g., (-)--(6S,6aR,9R,10aR)-5,6,6a,7,8,9,10,10a-octahydro-6-methyl-1-3-[(R)-1-methyl-4-phenylbutoxy]-1,9-phenanthridinediol 1-acetate)); certain aminoalkylindole analogs (e.g., (R)-(+)--[2,3-dihydro-5-methyl-3-(4-morpholinylm-ethyl)-pyrrolo[1,2,3,-de-]-1,4-benzoxazin-6-yl]-1-naphthelenyl-methanone); certain open pyran-ring analogs (e.g., 2-[3-methyl-6-(1-methylethenyl-2-cyclohexen-1-yl]-5-pentyl-1,3-benzendi-o-l, and 4-(1,1-dimethylheptyl)-2,3'-dihydroxy-6'-a-(3-hydroxypropyl)-1',-2'-,3',4',5',6'-hexahydrobiphenyl), their salts, solvates, metabolites, and metabolic precursors.

Cannabidiol can be obtained from plant extract, for example hemp, with a trace amount of delta-9-tetrahydrocannabinol or from cannabis extract using a high-cannabidiol cannabis cultivar. Further, cannabinoids are terpenophenolic compounds found in the Cannabis plant belonging to the Cannabaceae family. Cannabinoids can be isolated, for example, by extraction processes or cold pressing from cannabis plants. Plants in the cannabis genus include Cannabis sativa, Cannabis ruderalis, and Cannabis indica. These plants are the natural sources of cannabinoids, a preferred embodiment of this disclosure. Cannabinoids are also available in microbial-derived and synthetic forms. For example, altered brewer's yeast has been used to produce cannabinoids, Nature, "Scientists brew cannabis using hacked beer yeast", (27 February 2019). In another example, synthetic cannabinoids have been investigated: see, Sholler et al., "Therapeutic potential and safety considerations for the clinical use of synthetic cannabinoids", Pharmacol Biochem Behav. 2020 Dec;199:173059.

Examples of cannabinoids, cannabinoid analogs and cannabinoid derivatives include, but are not limited to, cannabigerolic acid (CBGA), cannabigerolic acid monomethylether (CBGAM), cannabigerol (CBG), cannabigerol monomethylether (CBGM), cannabigerovarinic acid (CBGVA), cannabigerovarin (CBGV), cannabichromenic acid (CBCA), cannabichromene (CBC), cannabichromevarinic acid (CBCVA), cannabichromevarin (CBCV), cannabidiolic acid (CBDA), cannabidiol (CBD), cannabidiol monomethylether (CBDM), cannabidiol-C.sub.4 (CBD-C.sub.4), cannabidivarinic acid (CBDVA), cannabidivarin (CBDV), cannabidiorcol (CBD-C.sub.1), delta-9-tetrahydrocannabinolic acid A (THCA-A), delta-9-tetrahydrocannabinolic acid B (THCA-B), delta-9-tetrahydrocannabinol (THC), delta-9-tetrahydrocannabinolic acid-C.sub.4 (THCA-C.sub.4), delta-9-tetrahydrocannabinol-C.sub.4 (THC-C.sub.4), delta-9-tetrahydrocannabivarinic acid (THCVA), delta-9-tetrahydrocannabivarin (THCV), delta-9-tetrahydrocannabiorcolic acid (THCA-C.sub.1), delta-9-tetrahydrocannabiorcol (THC-C.sub.1), delta-7-cis-iso-tetrahydrocannabivarin, delta-8-tetrahydrocannabinolic acid (.DELTA.sup.8-THCA), delta-8-tetrahydrocannabinol (.DELTA.sup.8-THC), cannabicyclolic acid (CBLA), cannabicyclol (CBL), cannabicyclovarin (CBLV), cannnabielsoic acid A (CBEA-A), cannabielsoic acid B (CBEA-B), cannabielsoin (CBE), cannabinolic acid (CBNA), cannabinol (CBN), cannabinol methylether (CBNM), cannabinol-C.sub.4 (CBN-C.sub.4), cannabivarin (CBV), cannabinol-C.sub.2 (CBN-C.sub.2), cannabiorcol (CBN-C.sub.1), cannabinodiol (CBND), cannabinodivarin (CBVD), cannabitriol (CBT), 10-ethoxy-9-hydroxy-delta-6a-tetrahydrocannabinol, 8,9-dihydroxy-delta-6a-tetrahydrocannabinol, cannabitriolvarin (CBTV), ethoxy-cannabitriolvarin (CB TVE), dehydrocannabifuran (DCBF), cannabifuran (CBF), cannabichromanon (CBCN), cannabicitran (CBT), 10-oxo-delta-6a-tetrahydrocannabinol (OTHC), delta-9-cis-tetrahydrocannabinol (cis-THC), 3,4,5,6-tetrahydro-7-hydroxy-alpha-alpha-2-trimethyl-9-n-propyl-2,6-metha- -no-2H-1-benzoxocin-5-methanol (OH-iso-HHCV), cannabiripsol (CBR) and trihydroxy-delta-9-tetrahydrocannabinol (triOH-THC).

In a particular embodiment of this disclosure, the cannabinoid is a naturally derived product. Naturally derived cannabinoids can be found in a Cannabis Sativa plant variety. Both CBD and THC are commercially available in large quantities. The art has shown that there are cannabinoid receptor sites in the human central nervous system and other tissues. These receptor sites, and their endogenous ligands, comprise the endocannabinoid system. CB 1 receptors have also been found in other mammalian tissue, such as in the heart, lung, pituitary glands, etc. CB2 receptor sites can be found in immune cells. The combination of CB1 and CB2 receptors can play an important role in regulating the release of 'messages' to the body, *i.e*., CB1 receptors from neurons and CB2 receptors from immune cells.

Cannabinoids are usually characterized by low solubility in water and high solubility in lipid mixtures. They do not easily adhere to or remain on skin, and have limited penetration properties. Given these limitations, it is challenging for a skin care formulator to find the best (inactive) co-ingredients, and the best loading requirements, to enhance the efficacy of a cannabinoid (an active ingredient) in the composition when it is applied to and comes in contact with the skin of a human or an animal.

Cannabinoids have anti-inflammatory attributes, which can also help to further moisturize, shrink, plump and smooth skin where needed. (CBD and CBD/THC) are natural plant-based compositions that are useful for treating acne and other skin disorders, while also providing skin rejuvenating properties. When a therapeutically effective amount of the composition is placed in contact with an area of a skin to be treated, the composition can treat a variety of disorders associated with skin ailments, conditions or diseases .

CBD and/or THC characteristics and properties are unexpectedly enhanced by combining it with meadowfoam seed oil and squalane, which serve as an emollient, penetration enhancer and/or a dispersion agent for the cannabinoid. The anti-microbial and skin metabolic enhancing properties of the embodied compositions make them exceptional dermal care or treatment products that can promote both skin healing and skin rejuvenation.

The topical compositions of this disclosure overcome some of the limitations in skin care products by developing a topical composition comprising lanolin and derivatives thereof, naturally derived cannabinoid(s), naturally derived vegetable oil(s), and naturally derived plant oil(s). One aspect of this disclosure is a formulation having (i) about 0.05-15 wt% of at least one cannabinoid and (ii) about 1-45 wt% of at least one vegetable oil and plant oil. Exemplary cannabinoids include CBD, THC, or CBD and THC. In one embodiment, the CBD comprises about 0.25-10% by weight of the total composition. In another embodiment, the composition comprises about 0.25-5% wt% of CBD and about 0.25-5 wt% of THC.

In particular embodiments, the plant oil comprises meadowfoam seed oil, squalane, or meadowfoam seed oil and squalane. In one formulation, the composition comprises about 5-20 wt% of meadowfoam seed oil and about 4-15 wt% of squalane. In another formulation, the composition comprises about 0.25-3 wt% of CBD, about 0.25-3 wt% by weight of THC, about 5-20 wt% of meadowfoam seed oil, and about 4-15 wt% of squalane.

In other embodiments, the composition further comprises one or more of the following ingredients: a solvent, a thickener, an anti-oxidant, a conditioner, a moisturizer, an emulsifier, a nutrient, an aroma, a preservative, a stabilizer, or a combination thereof.

The anti-microbial compositions and methods of use described herein are helpful for treating a variety of skin disorders, particularly, open wounds or broken, cracked skin conditions.

In embodiments, a topical composition of this disclosure may further comprise an inactive ingredient, such as a solvent, stabilizer, thickener, anti-oxidant, conditioner, moisturizer, preservative, nutrient, fragrance, cationic, ionic and non-ionic surfactant, antipruritic agent, antiperspirant, antipsoriatic agent, antiseborrheic agent, anti-aging agent, anti-wrinkle agent, skin lightening agent, depigmenting agent, or vitamin.

### Additional Embodiments

The topical composition may also further comprise one or more active ingredients, such as an antibiotic, an antiseptic agent, an antifungal, an antibacterial agent, an analgesic, an antiviral agent, an anesthetic, or an anti-cancer agent.

In embodiments, a topical composition can be in the form of a spray, an ointment, a cream, an emulsion, a lotion, a paste, an unguent, or a gel.

Creams according to this disclosure include water-in-oil or oil-in-water emulsions and may further comprise a cleansing agent, an emollient or an aromatic chemical compound.

Ointments and unguents according to this disclosure optionally contain oil and water that can be in a ratio from about 2:1 to 7:1, and may further comprise a wax, alcohol or petroleum-based mollifying agents.

Gels according to this disclosure optionally contain a vegetable oil up to 5% by weight of the total composition, water and a thickening agent. The thickening agent may be a natural polysaccharide, such as xanthan gum, carrageen, alginate or cellulose gum.

Pastes according to this disclosure may optionally contain aloe gel and beeswax.

Lotions according to this disclosure include oil-in-water or water-in-oil emulsions and may comprise cetyl alcohol, an emulsifier, a fragrance, glycerol, petroleum jelly, a dye, one or more preservatives and/or a stabilizing agent.

Exemplary antibiotics include, but are not limited to, ampicillin, bacampicillin, carbenicillin indanyl, mezlocillin, piperacillin, ticarcillin, amoxicillin-clavulanic acid, ampicillin-sulbactam, benzylpenicillin, cloxacillin, dicloxacillin, methicillin, oxacillin, penicillin G, penicillin V, piperacillin tazobactam, ticarcillin clavulanic acid, nafcillin, procaine penicillin, cefadroxil, cefazolin, cephalexin, cephalothin, cephapirin, cephradine, cefaclor, cefamandole, cefonicid, cefotetan, cefoxitin, cefprozil, cefmetazole, cefuroxime, loracarbef cefdinir, ceftibuten, cefoperazone, cefixime, cefotaxime, cefpodoxime, proxetil, ceftazidime, ceftizoxime, ceftriaxone, cefepime, azithromycin, clarithromycin, clindamycin, dirithromycin, erythromycin, lincomycin, troleandomycin, cinoxacin, ciprofloxacin, enoxacin, gatifloxacin, grepafloxacin, levofloxacin, lomefloxacin, moxifloxacin, nalidixic acid, norfloxacin, ofloxacin, sparfloxacin, trovafloxacin, oxolinic acid, gemifloxacin, perfloxacin, imipenem-cilastatin, meropenem, and aztreonam. In one embodiment, the amount of an antibiotic in a topical composition can be from about 0.01 to 5% by weight of the total composition.

Antiseptic compounds include, but are not limited to, iodine, manuka honey, octenidine dihydrochloride, phenol, polyhexanide, sodium chloride, sodium hypochlorite, calcium hypochlorite, sodium bicarbonate, methyl paraben, benzoyl peroxide and sodium dehydroacetate. In one embodiment, the amount of an antiseptic compound in the topical formulation can be from 0.01 to 5% by weight of the total composition.

Antifungal agents include, but are not limited to, amphotericin B, candicidin, filipin, hamycin, natamycin, nystatin, rimocidin, bifonazole, butoconazole, clotrimazole, econazole, fenticonazole, isoconazole, ketoconazole, luliconazole, miconazole, omoconazole, oxiconazole, sertaconazole, sulconazole, tioconazole, albaconazole, fluconazole, isavuconazole, itraconazole, posaconazole, ravuconazole, terconazole, voriconazole, abafungin, amorolfin, butenafine, naftifine, terbinafine, anidulafungin, caspofungin, micafungin, benzoic acid, ciclopirox, flucytosine, griseofulvin, haloprogin, tolnaftate, undecylenic acid, crystal violet, and balsam of Peru. In one embodiment, the amount of an antifungal agent in the topical formulation can be from 0.01 to 5% by weight of the total composition.

Analgesic agents include, but are not limited to, lidocaine, methyl salicylate, codeine, morphine, methadone, pethidine, buprenorphine, hydromorphine, levorphanol, oxycodone, fentanyl, and non-steroidal anti-inflammatory drugs. The amount of an analgesic agent in the topical formulation can be from about 0.01 to 5% by weight of the total composition.

Anti-viral agents include, but are not limited to, acyclovir, famciclovir, penciclovir, valacyclovir, trifluridine, docosanol, amantadine, rimantadine, oseltamivir, and zanamivir. The amount of an anti-viral agent in the topical formulation can be from about 0.01 to 5% by weight of the total composition.

In some embodiments, the topical composition of this disclosure may further comprise a stabilizer selected from the group consisting of guar gum, xanthan gum cellulose hyaluronic acid, polyvinyl pyrrolidone (PVP), alginate, chondroitin sulfate, poly gamma glutamic acid, gelatin, chitosan, corn starch and flour, in an amount from about 0.25 to 2% (w/v)

The art of formulating a skin care composition to treat skin disorders entails the sophisticated application of multiple scientific disciplines, e.g., chemistry, biology, physics, mathematics and materials. One of the common challenges to a formulator is how best to mix an active ingredient(s) with other ingredients (inactive or other active ingredients) to improve the efficacy of the composition. The use of one or more naturally derived cannabinoids, such as CBD, THC, or a combination of CBD and THC, together with a naturally derived meadowfoam seed oil and squalane, provides a desired beneficial topical formulation.

Formulations may be prepared by conventional procedures known in the pharmaceutical and cosmetic fields. It will be appreciated that the disclosed topical compositions may be varied according to well-known techniques to accommodate differing amounts and types of ingredients. Additionally, the specific components and ingredients and proportions described herein are for illustrative purposes. Ingredients may be exchanged for suitable equivalents or substitutes, and proportions may be varied, according to the desired properties of the dosage form of interest.

### Use and Efficacy

The use and efficacy of the described formulas can readily be evaluated by clinical trials, patient panel evaluations and user questionaries. User questionaries, for example, may provide data for a number of issue relevant to the compliancy and efficacy of the disclosed topical compositions. Data collected by questionaries can include, for example, user age, skin type, current skin care, location and cultural background. Additional data can include testing results, skin improvements, non-irritation, formulation appearance, application properties, fragrance and compliance. The data obtained from user questionaries provides useful information related to unexpected results, improved formulations, and beneficial treatment of skin and skin metabolism.

The following Examples are intended to illustrate the above disclosure and should not be construed as to narrow its scope. One skilled in the art will readily recognize that the Examples suggest many other ways in which this disclosure could be practiced. It should be understood that numerous variations and modifications may be made while remaining within the scope of this disclosure.

### EXAMPLES

### Sample 1 (CBD) - Toxicity and Metabolic Properties

A topical composition comprising CBD, meadowfoam seed oil and squalane, "Sample 1", included the following ingredients and weight percentages:
Aqua (Purified water) 57.20%, Limnanthes Alba (Meadowfoam) Seed Oil 15.00%, Neossance Saualane (Squalane) 10%, Cetearyl Alcohol (Plant) 5% and Cetearyl Glucoside and Sorbitan Olivate (Beautyderm K10) 5%, Olea Europaea (Olive) Oil Unsaponifiables 1%, Ethylhexylglycerin 1%, Caprylhydroxamic Acid and Glyceryl Caprylate and Glycerin 1.2%, Ascorbyl Palmitate and Tocopherol 1.00%, Cannabidiol (CBD) 0.55%, Sodium Hyaluronate 1.00% , Meadowfoam Delta Lactone 0.50%, Melatonin 0.050%, Glycine Soya (Soybean) Oil and Retinyl Palmitate and Tocopherol Acetate and Ascorbyl Isostearate 0.05%, Xanthan Gum 0.80%, Lavandula angustifolia (Lavender) Flower Oil 0.20%.

In addition to the ingredients and weight percentages, the following the general mixing conditions for making the topical composition, according to the primary functions of the ingredients, was used.

For example, in a suitable vessel, add solvent at room temperature, then slowly combine a thickener (add optional anti-oxidant) and mix with a LIGHTNIN mixer set at 600-800 rpm. This combined mixture is mixed for 10 minutes, and heated to 75-80 °C until completely dissolved and homogeneous still mixing at 600-800 rpm. In a separate suitable vessel, premix conditioner, moisturizer, emulsifier and cannabinoid(s) are mixed and heated to 80 °C with a LIGHTNIN mixer at 500-600 rpm. When both vessels are mixing and have reached between 75-80 °C, slowly transfer the premixed conditioner, moisturizer, emulsifier and cannabinoid(s) to the solvent and thickener while maintaining high mixing during and after the transfer. Maintain the 80 °C temperature while mixing at high speed for 5-10 minutes, then start slowing cooling the batch while reducing speed to 400-500 rpm. Cool batch to 55-60 °C and add premixed preservative treatment moisturizer and aroma therapy while mixing at this slower speed and continue to reduce speed as temperature drops. At 50 °C, submit a sample to QC for analysis and, if approved, transfer batch at between 40-50 °C for packaging.

Sample 1 was tested to determine its toxicity and metabolic properties in comparison to a control and three commercial cleaning agents (irritants) - "SDS" (sodium dodecyl sulfate) , Triton^{™} X-100, and Tween^{®} 20. Human skin was obtained via post-surgical elective surgery under IRB protocol and prepared within 2 hours of surgery as 5 mm explants. The compositions were applied to the surface of the epidermis and incubated for 24 hours and 48 hours at 37°C. At each time point, explants were assessed for viability and metabolic activity via a MTT assay (n = 3; mean +/- SEM), a colorimetric assay for assessing cell metabolic activity. After twenty-four hours post-exposure, Sample 1 exhibited enhanced metabolic activity beyond the metabolic activities of untreated *ex vivo* human skin explants, which were better than the metabolic activities shown by SDS, Triton^{®} X-100, and Tween^{®} 20. The metabolic activity (viability) of tissues treated with Sample 1 remained above 50% at 48 hours post treatment compared to the untreated skin control, while those treated with Trition^{®} X-100 and SDS were <10% and are illustrated in the graph below that plots percent viability and time.

The metabolic enhancement properties of Sample 1, when it comes in contact the skin, will promote skin rejuvenation properties, such as collagen rejuvenation, desquamation (increased epidermal turnover), and product application can accelerate the cell cycle with a faster turnover rate, yielding improvements in skin appearance.

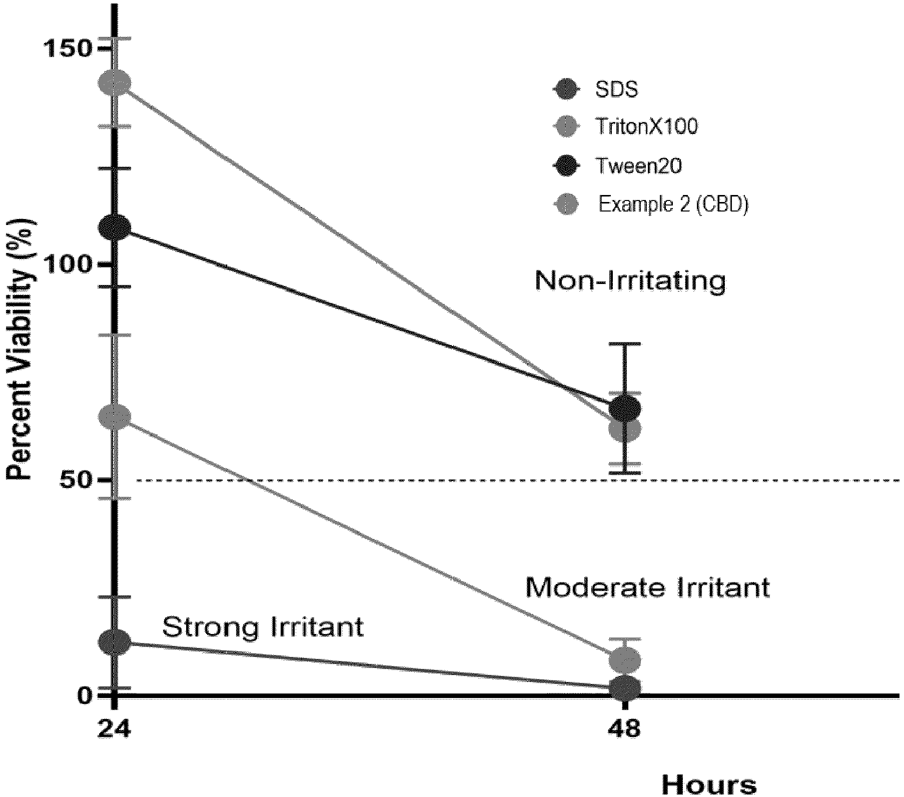

Specific embodiments of this disclosure are illustrative and do not limit the scope of this disclosure. Changes and modifications can be made in accordance with ordinary skill in the art without departing from this disclosure in its broader aspects as defined in the following claims.

No limitations inconsistent with this disclosure are to be understood therefrom. This disclosure has been described with reference to various specific and preferred embodiments and techniques. However, it should be understood that many variations and modifications may be made while remaining within the spirit and scope of this disclosure. All publications, patents, and patent documents are incorporated by reference herein, as though individually incorporated by reference.

## Claims

1. A topical composition comprising a combination of a pharmacologically effective amount of one or more extracted vegetable oils, one or more extracted plant oils, and one or more cannabinoids.

2. A topical composition comprising pharmacologically effective amounts of meadowfoam seed oil, squalane and cannabinoids.

3. The topical composition of claim 1 comprising about 1-20 wt% meadowfoam seed oil.

4. The topical composition of claim 1 comprising about 4-15 wt% meadowfoam seed oil.

5. The topical composition of claim 1 comprising about 5-7 wt% meadowfoam seed oil.

6. The topical composition of claim 1 comprising about 1-25 wt % squalane.

7. The topical composition of claim 1 comprising about 5-20 wt % squalane.

8. The topical composition of claim 1 comprising about 3-7 wt % squalane.

9. The topical composition of claim 1 comprising about 0.5-5 wt % squalane.

10. The topical composition of claim 1 comprising about 0.05-15 wt% cannabinoids.

11. The topical composition of claim 1 comprising about 0.25-10 wt% cannabinoids.

12. The topical composition of claim 1 comprising about 1-3 wt% cannabinoids.

13. The topical composition of claim 1 wherein the cannabinoids comprise THC.

14. The topical composition of claim 1 wherein the cannabinoids comprise CBD.

15. The topical composition of claim 1 wherein the CBD comprises about 0.25-5 wt% of the total composition.

16. The topical composition of claim 1 wherein the topical composition comprises about 0.25-5 wt% CBD and about 0.25-5 wt% THC.

17. The topical composition of claim 1 the topical composition comprises (i) about 0.05-15 wt% of at least one cannabinoid; and (ii) about 1-45 wt% of at least one extracted vegetable oil and one extracted plant oil.

18. The topical composition of claim 1 wherein the topical composition components comprise one of more of the following features:
(a) the cannabinoid, the extracted vegetable oil, and extracted plant oil comprise a naturally derived product;
(b) the cannabinoid comprises CBD, THC, or CBD and THC;
(c) the CBD comprises about 0.25-10 wt% of the total composition;
(d) the composition comprises about 0.25-5 wt% of CBD and about 0.25-5 wt% of THC;
(e) the extracted vegetable oil and extracted plant oil comprise meadowfoam seed oil and squalane;
(f) the composition comprises about 1-20 wt% of meadowfoam seed oil and about 1-25 wt% squalane; or
(g) the composition comprises about 0.25-5 wt% of CBD, about 0.25-5 wt% of THC, about 4-15 wt% of meadowfoam seed oil, and about 5-20 wt% of squalane.

19. The topical composition of claim 1 further comprising on or more of a solvent, a thickener, an anti-oxidant, a conditioner, a moisturizer, an emulsifier, a nutrient, an aroma or fragrance, a preservative, a stabilizer, or a combination thereof.

20. The topical composition of claim 1 comprising a spray, an ointment, a cream, an emulsion, a lotion, a paste, an unguent, or a gel.

21. The topical composition of claim 1 comprising a waterproof shield on skin.

22. A composition of claim 1 comprising a pharmacologically effective antimicrobial composition comprising pharmacologically effective amounts of meadowfoam seed oil, squalane and cannabinoids; wherein the cannabinoids topically penetrate and treat skin.

23. A method to topically treat skin comprising applying the topical composition of any one of claims 1-20 to a subject's skin, wherein the cannabinoids topically penetrate and treat skin ailments, conditions or diseases.
